# FASCICULE DE BREVET EUROPEEN

(11) **EP 2 544 637 B1**
(45) Date de publication et mention de la délivrance du brevet: **23.09.2015**
(21) Numéro de dépôt: 11717018.3
(22) Date de dépôt: 10.03.2011
(51) Int. Cl.: A61F 5/01, A61F 13/06

(54) **DISPOSITIF ORTHOPÉDIQUE POUR LE TRAITEMENT DE L'HALLUX VALGUS PAR EFFET MÉCANIQUE**
ORTHOPÄDISCHE VORRICHTUNG ZUR MECHANISCHEN BEHANDLUNG VON HALLUX VALGUS
ORTHOPEDIC DEVICE FOR THE MECHANICAL TREATMENT OF HALLUX VALGUS

(30) Priorité: 10.03.2010 FR 1000958
(43) Date de publication de la demande: 16.01.2013
(73) Titulaire: Sarl Pody Concept, 59110 La Madeleine (FR)
(72) Inventeur: FONTAINE, Thierry, 59155 Faches Thumesnil (FR); GANTIE, Cédric, 31870 Lagardelle Sur Leze (FR)
(74) Mandataire: de Roquemaurel, Bruno
(86) Numéro de dépôt international: PCT/IB2011/051013
(87) Numéro de publication internationale: WO 2011/111019

(56) Documents cités:
- WO-A1-2008/102405
- FR-A1- 2 695 028
- FR-A1- 2 892 298
- US-A- 5 497 789
- US-A1- 2003 005 601

## Description

L'invention concerne un dispositif orthopédique pour le traitement de l'Hallux Valgus par réaxation de l'Hallux Valgus par effet mécanique. Ce dispositif peut être porté le jour (chaussé) et la nuit.

L'Hallux Valgus est une déformation du pied caractérisée par l'inflexion latérale du gros orteil vers les autres orteils (mouvement d'abduction par rapport à l'axe du corps) avec un rapprochement du premier métatarsien de l'axe du corps (adduction) créant ainsi un angle augmenté entre le premier et le deuxième métatarsien. Ces deux composantes font que le premier métatarsien fait un angle avec la première phalange du gros orteil dont le sommet crée une saillie, zone généralement douloureuse et à l'origine de frottements.

Les personnes souffrant d'Hallux Valgus peuvent porter des chaussures larges et souples, des chaussures spécialisées ou encore des chaussures telles qu'enseignées, par exemple, dans le document FR 2.895.235. Si ces chaussures permettent indéniablement de soulager leurs porteurs, elles n'ont pas de fonction réparatrice de réaxation de l'Hallux Valgus.

Afin de corriger la déviation du gros orteil, il existe dans le commerce des séparateurs. Différents séparateurs existent sur le marché, à savoir : des séparateurs de conception paramédicale, réalisés sur mesure par un podologue ou de manière industrielle, ou encore des séparateurs qui écartent l'orteil de manière mécanique au travers de matériaux durs et rigides faits sous forme d'attelles rigides de nuit permettant, en outre de contraindre le premier métatarsien et une première réaxation mécanique par armatures. Dans tous les cas, ces dispositifs seront portés soit le jour chaussés (séparateurs) ou la nuit non chaussés (attelle avec armature rigide).

On connaît, par ailleurs, du document FR-2.576.209 un dispositif qui peut être porté chaussé permettant de maintenir en effectuant une traction légère et progressive donnant au gros orteil et aux autres orteils du pied la possibilité de reprendre leur axe.

Ce dispositif est constitué par une semelle orthopédique, qui est associée à une palette mobile permettant la traction du gros orteil à l'aide d'une lanière en cuir recouverte de peau qui se fixe au moyen d'un dispositif de fixation boucles/crochets, connu sous l'appellation « Velcro », soit sous la semelle en voûte plantaire, soit autour de la cheville.

Si cette semelle orthopédique, présentant l'encombrement d'une semelle, peut être portée chaussée, il est peu probable qu'elle convienne à tous les modèles de chaussures, plus particulièrement aux modèles étroits et serrés, tels qu'ils peuvent être portés par les femmes notamment. Par ailleurs, cette semelle constitue en quelque sorte une attelle rigide non agréable pour le porteur. En outre, le dispositif du document FR-2.576.209 ne peut être porté que pied nu, sans chaussette.

On connait par ailleurs, du document US 5 497 789 un dispositif comportant notamment un manchon élastique destiné à être porté sur le pied, et un coussinet assujetti sur la paroi interne du manchon de façon à être disposé latéralement au pied, contre la tête du métatarsien du gros orteil. Ce coussinet, réalisé en un matériau viscoélastique, est tel qu'il absorbe et dissipe par sa structure interne toute force latérale tangentielle appliquée à la tête du métatarsien du gros orteil, sans glisser par rapport à celle-ci.

Le but de la présente invention est de pallier les inconvénients précités en proposant un dispositif orthopédique pour le traitement de l'Hallux Valgus constituant une attelle fine, souple, non rigide, qui peut être portée chaussée quelle que soit la chaussure.

Un autre but de l'invention est de proposer un dispositif qui, par effet contensif ou de contention notamment, permet de soulager son porteur.

Un autre but de la présente invention est de proposer un tel dispositif qui n'entrave pas la dynamique du pied lors de la marche, mais qui au contraire, met à profit cette dynamique pour la réaxation de l'Hallux Valgus.

D'autres buts et avantages apparaîtront au cours de la description qui va suivre qui n'est donnée qu'à titre indicatif et qui n'a pas pour but de la limiter.

Aussi des modes de réalisation concernent un dispositif orthopédique pour le traitement de l'Hallux Valgus, le dispositif convenant pour être porté chaussé, constitué essentiellement par un manchon élastique destiné à être porté sur le pied pour exercer un effet de contention localisé sur les métatarsiens du pied, le manchon comprenant : une partie principale destinée à épouser le pied localement au niveau des métatarsiens du pied, une partie distale pour encapuchonner le gros orteil, une bande de jonction entre la partie principale et la partie distale, destinée à être positionnée latéralement au pied pour être sollicitée en traction afin d'exercer sur le gros orteil une force latérale dirigée vers l'intérieur par rapport au plan de symétrie du corps humain, et un coussinet assujetti sur la paroi interne du manchon élastique, créant une surépaisseur localisée, le coussinet étant agencé pour être positionné latéralement au pied, localement contre le premier métatarsien du gros orteil. Selon un mode de réalisation, le coussinet est réalisé en un matériau présentant un coefficient de frottement tel que le coussinet fait contact avec la peau du pied, sans glisser, sous la pression de l'effet de contention exercé par le manchon, de manière à servir d'ancrage à l'exercice de la force latérale, au moins une partie du coussinet étant apte à être disposée en différentes positions le long du premier métatarsien du gros orteil de telle sorte à pouvoir régler l'effort de traction sur la bande de jonction.

Selon un mode de réalisation, le coussinet présente une épaisseur comprise entre 2 mm et 7 mm, une longueur comprise entre 4 cm et 6 cm et une largeur comprise entre 2 cm et 4 cm.

Selon un mode de réalisation, le coussinet est fixé à l'intérieur du manchon sur la bande de liaison et sur la partie principale du manchon, le coussinet étant réalisé en un matériau élastique de manière à pouvoir être étiré et conserver son étirement par contact avec la peau du pied, sans glisser, afin de contribuer à l'exercice sur le gros orteil de la force latérale.

Selon un mode de réalisation, le matériau du coussinet est du néoprène ou du silicone, ou du gel de silicone.

Selon un mode de réalisation, le gel de silicone formant le matériau du coussinet en contact avec la peau comprend un produit actif apte à être transmis par contact à la peau.

Selon un mode de réalisation, le dispositif présente une partie proximale destinée à venir appuyer sur la partie arrière du pied pour assurer le maintien du manchon élastique au pied.

Selon un mode de réalisation, la partie proximale est réalisée par une boucle du manchon élastique, destinée à être positionnée autour de la cheville du pied.

Selon un mode de réalisation, le coussinet est assujetti au manchon élastique de manière permanente ou grâce à des moyens de fixation amovibles.

Selon un mode de réalisation, les moyens de fixation amovibles comprennent une pochette formée dans le manchon élastique, apte à recevoir de manière amovible le coussinet, ou comprennent un système de fixation à boucles et crochets entre le coussinet et le manchon élastique.

Selon un mode de réalisation, la partie distale est conformée de manière à encapuchonner le gros orteil sans être en contact avec la peau du gros orteil dans des régions de bord latéral de l'ongle du gros orteil, en l'absence de force de traction exercée sur la bande de jonction, afin de limiter la pression exercée par l'orthèse sur la peau du gros orteil dans les régions de bord latéral de l'ongle, en présence d'une force de traction exercée sur la bande de jonction.

Selon un mode de réalisation, le manchon élastique est réalisé en un textile élastique tel que l'élasthanne.

Selon un mode de réalisation, la partie distale et la bande de jonction sont réalisées en un textile élastique présentant une raideur plus faible que le textile formant la partie principale du manchon.

Selon un mode de réalisation, le manchon élastique est constitué à partir d'un patron textile après des opérations de couture.

Selon un mode de réalisation, le manchon élastique présente une épaisseur inférieure à 1 mm.

L'invention sera mieux comprise à la lecture suivante accompagnée des dessins en annexe parmi lesquels:
- la figure 1 est une vue d'un dispositif, selon un mode de réalisation, non porté,
- la figure 2 est une vue d'un pied sur lequel le dispositif va être porté,
- les figures 3 à 10 illustrent différentes étapes de mise en place du dispositif tel qu'illustré à la figure 1, sur le pied tel qu'illustré à la figure 2, et plus particulièrement :

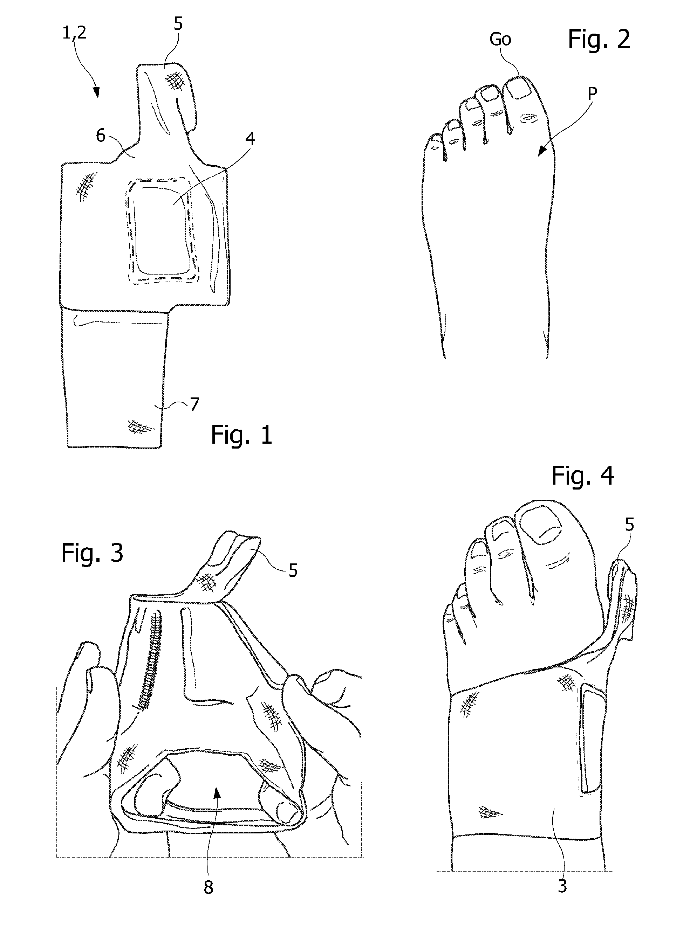

- la figure 3 représente le dispositif dans une configuration écartée à l'aide des mains pour permettre le passage du pied,
- la figure 4 illustre la mise en place d'une partie principale du dispositif sur le pied autour des métatarsiens,

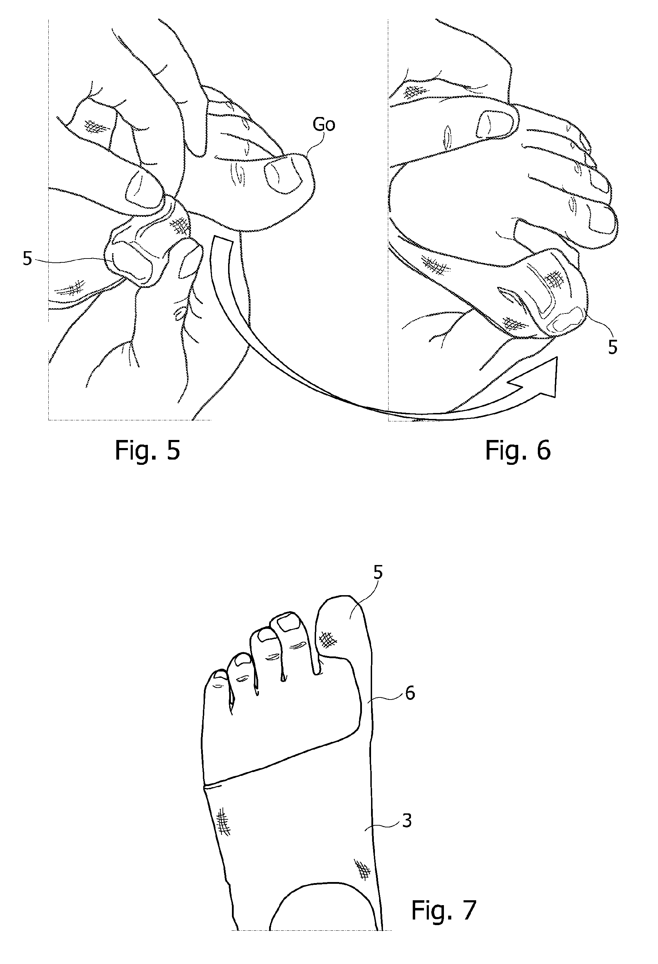

- les figures 5 et 6 illustrent l'encapuchonnage du gros orteil par une partie distale du dispositif,
- la figure 7 est une vue partielle du dispositif sur l'avant du pied lorsqu'il est correctement porté,

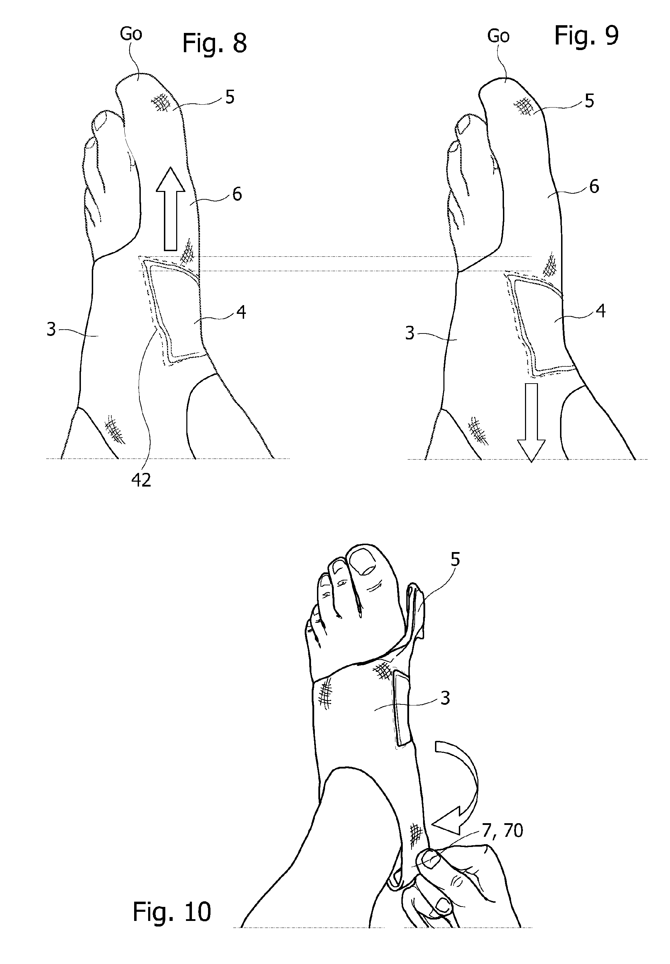

- la figure 8 illustre une étape dans laquelle un effort de traction exercé par le dispositif est diminué,
- la figure 9 est une vue dans laquelle l'effort de traction exercé par le dispositif est augmenté,
- la figure 10 illustre la mise en place d'une partie proximale du dispositif autour de la cheville,

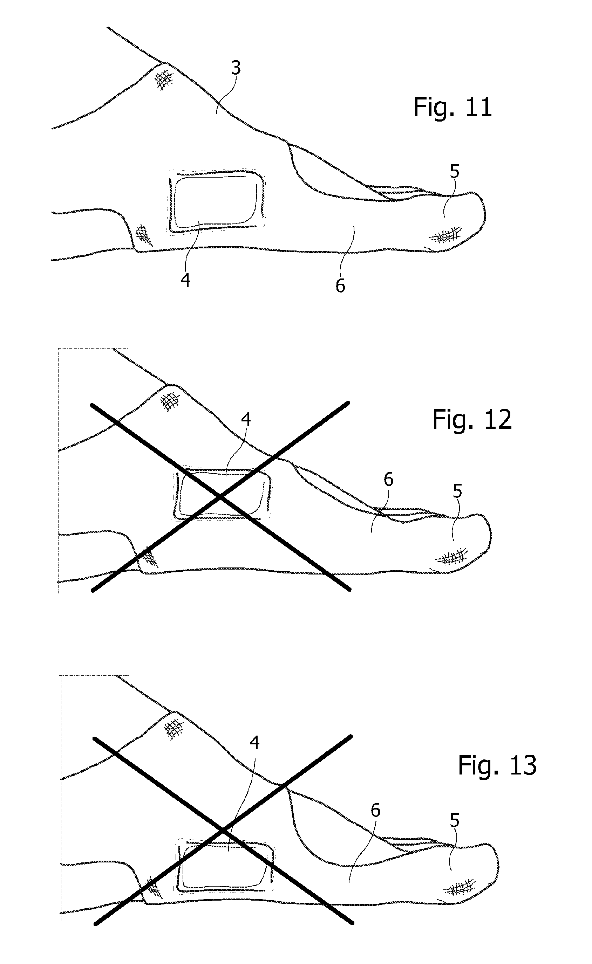

- la figure 11 est une vue latérale intérieure du dispositif lorsqu'il est correctement porté sur le pied,
- la figure 12 est une vue latérale intérieure du dispositif lorsqu'il est porté sur le pied dans une configuration trop haute,
- la figure 13 est une vue latérale intérieure du dispositif lorsqu'il est porté sur le pied dans une configuration trop basse,

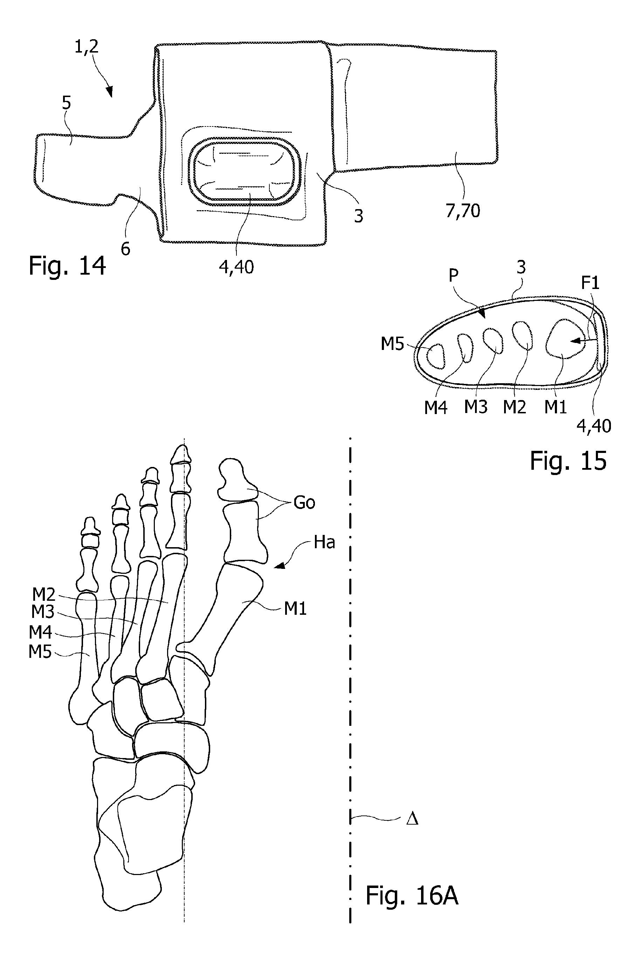

- la figure 14 est une vue du dispositif, à l'état retroussé, permettant de visualiser un coussinet interne,
- la figure 15 est une vue en coupe frontale au niveau de la palette métatarsienne du dispositif porté sur le pied, illustrant la coopération entre le coussinet et le premier métatarsien du gros orteil,

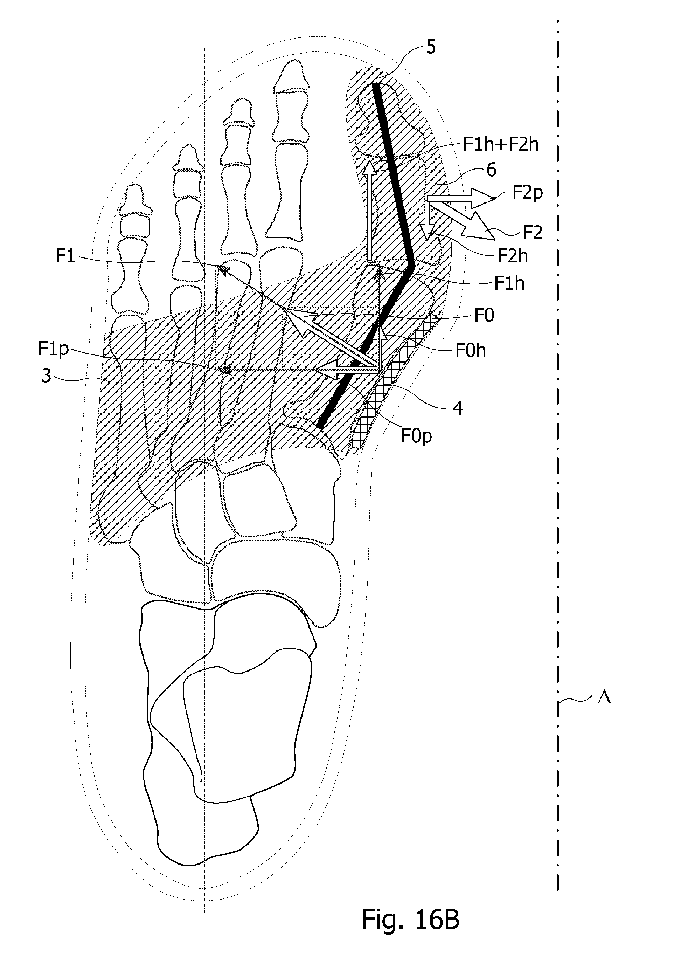

- les figures 16A, 16B sont deux vues de dessus de l'ossature d'un pied souffrant d'Hallux Valgus, respectivement sans et avec le dispositif,

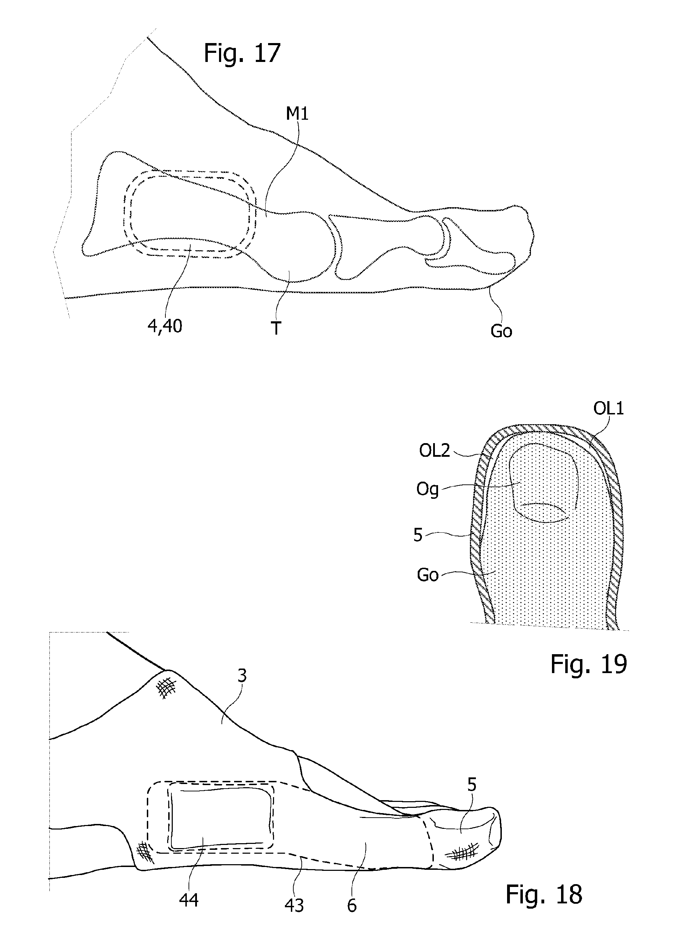

- la figure 17 est une vue latérale en coupe illustrant par transparence le positionnement idéal (en pointillés) du coussinet par rapport au premier métatarsien du gros orteil,
- la figure 18 représente un dispositif orthopédique selon un autre mode de réalisation,
- la figure 19 représente en coupe la partie distale du dispositif, selon un mode de réalisation.

Dans la présente invention, les termes « intérieur» et « extérieur» pour qualifier le sens de forces notamment, ont pour référentiel l'axe Δ du corps humain (voir figures 16A, 16B). L'axe Δ du corps humain est matérialisé par le plan de symétrie du corps humain. Lorsque la force est dirigée vers cet axe Δ, on dit qu'elle est dirigée vers l'intérieur. Lorsque la force est dirigée dans l'autre sens et s'écarte de l'axe Δ, on dit qu'elle est dirigée vers l'extérieur.

Les figures 1 et 16B représentent un dispositif 1 orthopédique pour le traitement de l'Hallux Valgus Ha, constitué essentiellement par un manchon élastique 2 destiné à être porté sur le pied P pour exercer un effet de contention localisé sur les métatarsiens M1, M2, M3, M4, M5 du pied (figure 16A). Cet effet de contention permet notamment de limiter l'adduction du premier métatarsien M1 par rapport à l'axe Δ du corps humain. Le dispositif 1 est souple et non rigide.

Selon un mode de réalisation, l'effet de contention localisé permet d'exercer sur le premier métatarsien M1 du gros orteil une force latérale F1 dirigée vers l'extérieur par rapport à l'axe Δ du corps humain, c'est-à-dire vers l'intérieur du pied, de manière à obtenir une réaxation du métatarsus varus et donc de l'Hallux Valgus.

Le dispositif 1 se présente sous la forme d'une attelle fine, d'épaisseur sensiblement égale à celle du matériau du manchon, telle que par exemple inférieure à 1 mm, produisant un effet de contention léger, soulageant l'utilisateur, et pouvant être portée en permanence, quel que soit l'article chaussant. Le dispositif 1 n'étant pas plus encombrant qu'une chaussette peu épaisse, il peut être porté avec tout type d'article chaussant, en dessous de bas ou chaussettes notamment.

Le dispositif 1 comprend un manchon élastique 2 ayant pour fonction première de rapprocher le premier métatarsien M1 du gros orteil vers l'axe du pied (et donc d'écarter le premier métatarsien M1 de l'axe Δ du corps humain). Cette action mécanique passive est permise au moins par l'élasticité du manchon 2 et a pour but de lutter contre le métatarsus varus associé au valgus du gros orteil.

Le manchon élastique 2 peut être réalisé à partir d'un textile. Il peut être constitué à partir d'un patron, notamment selon la pointure du pied, après des opérations de couture. Le textile du manchon souple élastique 2 peut comprendre des fibres d'élasthanne conférant au textile son élasticité. Par exemple, le textile peut être en Lycra (marque déposée).

Le dispositif 1 comprend un coussinet 4, sur la paroi interne du manchon élastique 2, créant une surépaisseur localisée destinée à être positionnée latéralement au pied, localement contre le premier métatarsien M1 du gros orteil, et tel qu'illustré aux figures 15, 16B et 17.

Avantageusement, le coussinet 4 appuie sur le premier métatarsien M1 et diminue l'adduction de celui-ci de manière progressive lors de la poussée valgisante du pied afin de le propulser dans l'axe sur le gros orteil.

Tel qu'illustré à la figure 17, le dimensionnement du coussinet 4 (en pointillés) correspond sensiblement aux dimensions du premier métatarsien M1. La longueur du coussinet 4 est légèrement inférieure à la longueur de la diaphyse du premier métatarsien, de manière à autoriser un positionnement du coussinet 4 en différentes positions le long de la diaphyse du premier métatarsien, sans recouvrir la tête T de celui-ci.

Lorsque le dispositif est chaussé, le coussinet 4 est calé contre la paroi interne de la chaussure, augmentant ainsi la force latérale F1, pour la réaxation du premier métatarsien M1 du gros orteil.

Avantageusement, tel qu'illustré à la figure 17, le coussinet 4 ne recouvre pas la tête T du premier métatarsien M1 afin de décharger la saillie de l'Hallux Valgus, qui sinon, est bien souvent en appui contre la paroi interne de la chaussure.

Selon un mode de réalisation, le dispositif permet d'exercer sur le gros orteil Go, au moins indirectement, une force latérale F2 dirigée vers l'intérieur par rapport à l'axe Δ du corps humain, c'est-à-dire vers l'extérieur du pied, par appui du gros orteil sur l'orteil voisin.

Selon un mode de réalisation illustré par les figures 1 et 4, le manchon élastique 2 comprend, outre une partie principale 3 destinée à épouser de manière élastique le pied localement au niveau des métatarsiens M1, M2, M3, M4, M5 dudit pied en les enserrant (par ceinturage) pour exercer sur le premier métatarsien M1 du gros orteil Go la force latérale F1 dirigée vers l'extérieur par rapport à l'axe Δ du corps humain, une partie distale 5 pour encapuchonner le gros orteil Go ainsi qu'une bande de jonction 6 entre la partie principale 3 et la partie distale 5.

La partie distale 5 assure le maintien de la bande de jonction 6 latéralement le long du gros orteil Go pour lui permettre d'être sollicitée en traction afin d'augmenter la force latérale F2 dirigée vers l'intérieur par rapport à l'axe 0 du corps humain.

Les figures 3 à 6 illustrent différentes étapes de mise en place du dispositif 1. Sur la figure 3, le manchon élastique 2 est écarté à l'aide des mains pour élargir le passage 8 du pied dans le manchon et ainsi permettre l'introduction de l'avant du pied dans le manchon. Sur la figure 4, le manchon 2 est positionné de manière à entourer les métatarsiens M1-M5, et ainsi leur appliquer un effort de contention. Sur la figure 5, la partie distale 5 formant un capuchon, est tirée vers l'extrémité du gros orteil Go. Sur la figure 6, la partie distale 5 est engagée sur le gros orteil Go. Sur la figure 7, le dispositif 1 apparaît dans une position finale sur le pied.

Comme illustrée à la figure 11, la bande de jonction 6 doit être positionnée latéralement le long du gros orteil Go et du premier métatarsien dans l'axe du gros orteil, et non pas dans une position trop haute telle qu'illustrée à la figure 12, ou encore trop basse telle qu'illustrée à la figure 13.

Avantageusement, le dispositif peut présenter en outre des moyens de réglage de la force latérale F2 exercée sur le gros orteil Go. Les moyens de réglage de la force F2 peuvent être constitués par le coussinet 4 précédemment décrit. A cet effet, le coussinet 4 est réalisé en un matériau 40 présentant un coefficient de frottement suffisant pour faire contact directement avec la peau du pied, sans glisser. En d'autres termes, le coussinet 4 adhère à la peau. Cette adhésion peut être rendue possible par la force de contention exercée par la partie 3 du manchon 2, qui s'exerce aussi sur le coussinet 4.

Ainsi, tel qu'illustré selon les exemples des figures 8 ou 9, le coussinet 4 peut être positionné en différentes positions du pied de telle sorte à pouvoir régler l'effort de traction sur la bande de jonction 6. Sur les figures 8 et 9, la position du coussinet est visible par les coutures 42 solidarisant le matériau, notamment textile, du manchon 2 élastique sur le pourtour du coussinet.

Tel qu'illustré à la figure 8, le coussinet 4 est rapproché vers le gros orteil 40, afin de diminuer l'effort de traction sur la bande de jonction 6, et ainsi diminuer la force latérale F2 sur le gros orteil. Au contraire, tel qu'illustré à la figure 9, le coussinet 4 est éloigné du gros orteil Go afin d'augmenter l'effort de traction sur la bande de jonction 6 et donc la tension de la bande de jonction, et ainsi, augmenter la force latérale F2 exercée sur le gros orteil. L'ajustement de la position du coussinet 4 le long du premier métatarsien M1 permet ainsi de régler la tension de la bande de jonction 6.

Tel qu'illustré à la figure 16B, le manchon 2, en serrant les cinq métatarses M1-M5, exerce une force F0 qui se décompose suivant des axes parallèle à l'axe du pied et perpendiculaire à l'axe du pied, en une composante de force axiale F0h et une composante de force latérale F0p. La présence du coussinet 4 accentue la force F0, notamment pendant la marche et lorsque le pied est chaussé. La force résultante correspond à la force F1 précédemment mentionnée. La force F1 peut également être décomposée en une composante axiale F1h et une composante latérale F1p.

Par ailleurs, lorsque l'utilisateur déplace le coussinet 4 vers le talon en tirant sur la bande de jonction 6, cela crée la force F2 qui peut également se décomposer en une composante axiale F2h et une composante latérale F2p. Dans ces conditions, le gros orteil GO et le premier métatarsien M1 subissent une force résultante égale à F1h+F2h, la composante F2h étant réglable. Par ailleurs, les composantes F1p et F2p agissent en combinaison dans le sens d'une réduction l'Hallux Valgus, la composante F2p étant réglable.

Durant la marche, il peut être constaté que la composante F1p de la force F1 augmente à chaque déroulé du pas lors de la poussée exercée par le pied, et que cette composante diminue lorsque le pied quitte le sol. Les os qui convergent vers l'articulation (métatarse M1 et phalange du gros orteil) sont soumis par le dispositif 2, à des moments de forces variables tendant à réduire l'hallux valgus. Les mouvements qui en résultent sont de même nature que ceux que pourraient pratiquer un kinésithérapeute et se produisent de manière naturelle et douce pendant la marche.

Selon un mode de réalisation, le matériau 40 peut être, par exemple, du néoprène ou élastomère de silicone ou encore du gel de silicone. Le coussinet 4 est par exemple constitué d'une bande d'épaisseur comprise entre 2 et 7 mm par exemple, de longueur comprise entre 4 et 6 cm et de largeur comprise entre 2 et 4 cm.

Le coussinet 4 peut être assujetti de manière permanente au manchon élastique 2 (par exemple par couture, collage, ...). Alternativement, afin de pouvoir laver le matériau, notamment textile du manchon élastique 2, le coussinet 4 peut être séparé du manchon 2 en prévoyant des moyens de fixation amovibles (non illustrés) entre le coussinet 4 et le manchon élastique 2.

Par exemple, les moyens de fixation amovibles peuvent comprendre une pochette apte à recevoir de manière amovible le coussinet 4. Cette pochette peut présenter une fenêtre en regard du pied, afin d'autoriser le contact direct entre le matériau 40 non glissant (moyen de réglage) du coussinet 4 et le pied P. Alternativement, les moyens de fixation amovibles peuvent être constitués par un système de fixation à boucles et à crochets entre le coussinet 4 et le manchon élastique 2.

Le caractère amovible du coussinet 4 peut être avantageusement exploité pour utiliser des coussinets d'épaisseurs différentes, ceci afin d'ajuster la force F1 (et donc la composante F1p) exercée sur le premier métatarsien M1 en fonction de l'ampleur du métatarsus varus, et/ou en fonction de l'évolution de cette ampleur, y compris en cas de réduction. En effet, il est bien connu que la souplesse d'une articulation dépend largement de l'apprentissage dont elle bénéficie. On peut ainsi observer une tolérance de plus en plus grande aux forces correctrices au fur et à mesure de l'usage de l'orthèse, ce qui peut justifier l'usage d'épaisseurs de plus en plus grandes.

Dans un mode de réalisation illustré aux figures 1, 7 et 14, le dispositif peut en outre comprendre une partie proximale 7 destinée à prendre appui sur la partie arrière du pied P pour assurer le maintien dudit manchon élastique 2 sur le pied P. La partie proximale 7 est réalisée par une boucle 70 formée par le manchon élastique, destinée à être positionnée autour de la cheville du pied P, tel que cela est illustré à la figure 5.

Dans un mode de réalisation illustré à la figure 18, le coussinet 4 est remplacé par un coussinet 43 en forme de bande élastique. Le coussinet 43 est fixé à l'intérieur du manchon 2 à l'emplacement du coussinet 4, sur la bande de jonction 6, jusqu'à la partie distale 5. La bande 43 présente un coefficient de frottement avec la peau tel qu'elle peut être étirée et conserver son étirement par contact avec la peau du pied sans glisser, En tirant sur La partie 3 du manchon 2 dans une direction proximale pour l'écarter de la partie distale 5, la bande 43 s'étire avec la bande de liaison 6 et la partie 3 et reste dans sa configuration finale d'étirement en raison de son adhérence à la peau. Ainsi, la bande élastique 43 assure à la fois les fonctions d'augmenter par son épaisseur, la force de contention F1 exercée sur le métatarsien M1, d'ancrage par son coefficient de frottement avec la peau, pour l'exercice et l'ajustement de la force F2, et de contribuer par son élasticité avec la bande de jonction 6 et la partie 3 du manchon 2, à l'amplitude de la force F2. Le coussinet 43 peut être fixé à la partie 3 du manchon 2 et à la bande de jonction 6, par exemple par des coutures, ou par collage.

Dans un mode de réalisation, les fonctions de coussinet et d'ancrage pour l'exercice de la force F2 sont dissociées en prévoyant que le coussinet 43 fixé à l'intérieur du manchon 2 présente une épaisseur faible, et un coussinet supplémentaire 44 fixé entre le coussinet 43 et la partie 3 du manchon 2. Le coussinet 44 peut être identique au coussinet 4.

Dans un mode de réalisation, le coussinet 43 et/ou 44 est réalisé en néoprène ou en élastomère de silicone, ou encore en un gel polymère tel qu'un gel de silicone.

Dans un mode de réalisation illustré à la figure 19, la partie distale 5 est conformée de manière à ne pas exactement épouser la forme du gros orteil Go, et à ne pas venir en contact avec la peau du gros orteil dans des régions de bords latéraux OL1, OL2 de l'ongle Og du gros orteil Go (en l'absence de force de traction exercée sur la bande de jonction 6). Cette disposition permet de limiter la pression exercée par l'orthèse, en présence d'une force de traction exercée sur la bande de jonction 6, sur la peau dans les régions de bords latéraux de l'ongle du gros orteil, et ainsi d'augmenter le confort de l'utilisateur.

Dans un mode de réalisation, le manchon 2 est réalisé dans un tissu différent de celui dans lequel sont formés la bande de liaison et la partie distale 5. Ainsi, le tissu élastique dans lequel est formé le manchon 2 peut être plus raide que celui dans lequel sont formées la bande 6 et la partie distale 5, pour obtenir un effet de contention important sans provoquer de gêne dans la région du gros orteil qui est plus sensible.

Il est à noter que la raideur plus faible de la bande de jonction 6 peut être compensée par la présence de la bande élastique 43. La coopération entre la bande de jonction 6 et la bande élastique joue aussi pour la tenue dans le temps de la fonction élastique. En effet, les tissus ont toujours tendance à ne pas reprendre exactement leur longueur initiale après étirement, ce qui n'est pas le cas des bandes en gel de silicone utilisées. En l'absence de la bande 43, pour la même force la position du coussinet 4 se déplacerait vers le talon au point de perdre sa position utile, juste derrière l'articulation.

Dans un autre mode de réalisation, avec ou sans la bande élastique 43, le manchon peut être réalisé en une seule pièce de tissu présentant une raideur plus faible dans le sens de l'extension de la bande de jonction 6, que dans le sens de l'élargissement de la partie principale 3 pour exercer une force de contention sur les métatarsiens importante, sans gêne dans la région de l'extrémité du gros orteil.

Dans un mode de réalisation, la propriété des gels de silicone d'absorption de produits huileux peut être exploitée pour diffuser des produits actifs dans la peau. Ainsi, le coussinet 4 ou la bande 43 peut renfermer un produit actif à diffuser par contact à la peau. Le produit actif peut être choisi par exemple pour soulager la douleur ou soigner. Le produit actif peut être introduit dans le gel de silicone à plusieurs reprises, simplement en le versant sur le coussinet 4 ou la bande élastique 43.

Naturellement, d'autres modes de réalisation auraient pu être envisagés par l'homme du métier sans pour autant sortir du cadre de l'invention définie par les revendications ci-après.

## Revendications

1. Dispositif (1 ) orthopédique pour le traitement de l'Hallux Valgus (Ha), le dispositif convenant pour être porté chaussé, constitué essentiellement par un manchon (2) élastique destiné à être porté sur le pied (P) pour exercer un effet de contention localisé sur les métatarsiens (M1, M2, M3, M4, M5) du pied (P), le manchon comprenant :
une partie principale (3) destinée à épouser le pied (P) localement au niveau des métatarsiens (M1, M2, M3, M4, M5) du pied,
une partie distale (5) pour encapuchonner le gros orteil (Go),
une bande de jonction (6) entre la partie principale (3) et la partie distale (5), destinée à être positionnée latéralement au pied pour être sollicitée en traction afin d'exercer sur le gros orteil (Go) une force latérale (F2) dirigée vers l'intérieur par rapport au plan de symétrie du corps humain, et
un coussinet (4) assujetti sur la paroi interne du manchon élastique (2), créant une surépaisseur localisée, le coussinet étant agencé pour être positionné latéralement au pied, localement contre le premier métatarsien du gros orteil (Go),
**caractérisé en ce que** le coussinet (4, 43) est réalisé en un matériau (40) présentant un coefficient de frottement tel que le coussinet fait contact avec la peau du pied, sans glisser, sous la pression de l'effet de contention exercé par le manchon (2), de manière à servir d'ancrage à l'exercice de la force latérale (F2), au moins une partie du coussinet étant apte à être disposée en différentes positions le long du premier métatarsien du gros orteil (Go) de telle sorte à pouvoir régler l'effort de traction sur la bande de jonction (6).

2. Dispositif selon la revendication 1, dans lequel le coussinet (4, 43) présente une épaisseur comprise entre 2 mm et 7 mm, une longueur comprise entre 4 cm et 6 cm et une largeur comprise entre 2 cm et 4 cm.

3. Dispositif selon l'une des revendications 1 et 2, dans lequel le coussinet (43) est fixé à l'intérieur du manchon sur la bande de liaison (6) et sur la partie principale (3) du manchon (2), le coussinet (43) étant réalisé en un matériau élastique de manière à pouvoir être étiré et conserver son étirement par contact avec la peau du pied, sans glisser, afin de contribuer à l'exercice sur le gros orteil (Go) de la force latérale (F2).

4. Dispositif selon l'une des revendications 1 à 3, dans lequel le matériau du coussinet (4, 43, 44) est du néoprène ou du silicone, ou du gel de silicone.

5. Dispositif selon la revendication 4, dans lequel le gel de silicone formant le matériau du coussinet (4, 43) en contact avec la peau comprend un produit actif apte à être transmis par contact à la peau.

6. Dispositif selon l'une des revendications 1 à 5, présentant une partie proximale (7) destinée à venir appuyer sur la partie arrière du pied (P) pour assurer le maintien du manchon élastique (2) au pied.

7. Dispositif selon la revendication 6 dans lequel la partie proximale (7) est réalisée par une boucle (70) du manchon élastique (2), destinée à être positionnée autour de la cheville du pied (P).

8. Dispositif selon l'une des revendications 1 à 7, dans lequel le coussinet (4) est assujetti au manchon élastique (2) de manière permanente ou grâce à des moyens de fixation amovibles.

9. Dispositif selon la revendication 8, dans lequel les moyens de fixation amovibles comprennent une pochette formée dans le manchon élastique (2), apte à recevoir de manière amovible le coussinet (4), ou comprennent un système de fixation à boucles et crochets entre le coussinet (4) et le manchon élastique.

10. Dispositif selon l'une des revendications 1 à 9, dans lequel la partie distale (5) est conformée de manière à encapuchonner le gros orteil sans être en contact avec la peau du gros orteil dans des régions de bord latéral (OL1, OL2) de l'ongle du gros orteil, en l'absence de force de traction exercée sur la bande de jonction (6), afin de limiter la pression exercée par l'orthèse sur la peau du gros orteil dans les régions de bord latéral de l'ongle, en présence d'une force de traction exercée sur la bande de jonction (6).

11. Dispositif selon l'une des revendications 1 à 10, dans lequel le manchon élastique (2) est réalisé en un textile élastique tel que l'élasthanne.

12. Dispositif selon l'une des revendications 1 à 11, dans lequel la partie distale (5) et la bande de jonction (6) sont réalisées en un textile élastique présentant une raideur plus faible que le textile formant la partie principale (3) du manchon (2).

13. Dispositif selon la revendication 12, dans lequel le manchon élastique (2) est constitué à partir d'un patron textile après des opérations de couture.

14. Dispositif selon l'une des revendications 1 à 13, dans lequel le manchon élastique (2) présente une épaisseur inférieure à 1 mm.

## Patentansprüche

1. Orthopädische Vorrichtung (1) zur Behandlung des Hallux Valgus (Ha), wobei die Vorrichtung geeignet ist, mit Schuhen getragen zu werden, bestehend im Wesentlichen aus einer elastischen Hülse (2), die am Fuß (P) getragen wird, um eine an den Mittelfußknochen (M1, M2, M3, M4, M5) des Fußes (P) lokalisierte Stützwirkung auszuüben, die Hülse umfassend:
einen Hauptteil (3), der vorgesehen ist, um sich dem Fuß (P) lokal an den Mittelfußknochen (M1, M2, M3, M4, M5) des Fußes anzupassen,
einen distalen Teil (5), um die Großzehe (Go) einzuhüllen,
ein Verbindungsband (6) zwischen dem Hauptteil (3) und dem distalen Teil (5), das lateral zum Fuß angeordnet wird, um auf Zug beansprucht zu werden, um auf die Großzehe (Go) eine Querkraft (F2) auszuüben, die im Verhältnis zur Symmetrieebene des menschlichen Körpers nach Innen gerichtet ist, und
ein Kissen (4), das an der Innenseite der elastischen Hülse (2) angebracht ist und somit eine lokalisierte Verdickung bildet, wobei das Kissen angeordnet ist, um lateral zum Fuß, lokal gegen den ersten Mittelfußknochen der Großzehe (Go) positioniert zu werden,
**dadurch gekennzeichnet, dass** das Kissen (4, 43) aus einem Material (40) mit einem derartigen Reibungskoeffizienten realisiert ist, dass das Kissen unter dem Druck der von der Hülse (2) ausgeübten Stützwirkung in Kontakt mit der Fußhaut steht ohne zu rutschen, so dass es als Anker für die Ausübung der Querkraft (F2) dient, wobei zumindest ein Teil des Kissens geeignet ist, in verschiedenen Positionen am ersten Mittelfußknochen der Großzehe (Go) entlang angeordnet zu werden, so dass die Zugkraft am Verbindungsband (6) eingestellt werden kann.

2. Vorrichtung nach Anspruch 1, bei der das Kissen (4, 43) eine Dicke zwischen 2 mm und 7 mm, eine Länge zwischen 4 cm und 6 cm und eine Breite zwischen 2 cm und 4 cm aufweist.

3. Vorrichtung nach einem der Ansprüche 1 und 2, bei der das Kissen (43) im Inneren der Hülse am Verbindungsband (6) und am Hauptteil (3) der Hülse (2) befestigt ist, wobei das Kissen (43) aus einem elastischen Material realisiert ist, um gestreckt werden zu können und seine Streckung durch den Kontakt mit der Haut des Fußes bewahren zu können ohne zu rutschen, um der Ausübung der Querkraft (F2) auf die Großzehe (Go) beizutragen.

4. Vorrichtung nach einem der Ansprüche 1 bis 3, bei der das Material des Kissens (4, 43, 44) Neopren oder Silikon, oder Silikongel ist.

5. Vorrichtung nach Anspruch 4, bei der das Silikongel, das das Material des im Kontakt mit der Haut stehenden Kissens (4, 43) bildet, einen Wirkstoff umfasst, der geeignet ist, durch Kontakt an die Haut übertragen zu werden.

6. Vorrichtung nach einem der Ansprüche 1 bis 5 mit einem proximalen Teil (7), der vorgesehen ist, um sich auf den hinteren Teil des Fußes (P) so zu stützen, dass der Halt der elastischen Hülse (2) am Fuß gewährleistet wird.

7. Vorrichtung nach Anspruch 6, bei der der proximale Teil (7) durch eine Schlaufe (70) der elastischen Hülse (2) realisiert ist, die vorgesehen ist, um um den Knöchel des Fußes (P) positioniert zu werden.

8. Vorrichtung nach einem der Ansprüche 1 bis 7, bei der das Kissen (4) permanent oder mit Hilfe von entfernbaren Befestigungsmitteln an der elastischen Hülse (2) angebracht ist.

9. Vorrichtung nach Anspruch 8, bei der die entfernbaren Befestigungsmittel ein in der elastischen Hülse (2) gebildetes Täschchen umfassen, das geeignet ist, auf entfernbare Art das Kissen (4) aufnehmen zu können, oder ein Befestigungssystem mit Schlaufen und Haken zwischen dem Kissen (4) und der elastischen Hülse umfassen.

10. Vorrichtung nach einem der Ansprüche 1 bis 9, bei der der distale Teil (5) derart ausgebildet ist, dass er die Großzehe einhüllt, ohne in Abwesenheit einer auf das Verbindungsband (6) ausgeübten Zugkraft in Kontakt mit der Haut der Großzehe in Seitenrandgebieten (OL1, OL2) des Fußnagels der Großzehe zu stehen, um den von der Orthese auf die Haut der Großzehe in den Seitenrandgebieten des Fußnagels, in Anwesenheit einer auf das Verbindungsband (6) ausgeübten Zugkraft, ausgeübten Druck zu begrenzen.

11. Vorrichtung nach einem der Ansprüche 1 bis 10, bei der die elastische Hülse (2) aus einem elastischen Textilmaterial wie Elastan realisiert ist.

12. Vorrichtung nach einem der Ansprüche 1 bis 11, bei der der distale Teil (5) und das Verbindungsband (6) aus einem elastischen Textilmaterial mit einer geringeren Steifigkeit als das den Hauptteil (3) der Hülse (2) bildende Textilmaterial realisiert sind.

13. Vorrichtung nach Anspruch 12, bei der die elastische Hülse (2) nach einem Textilmuster nach Näharbeiten gebildet wird.

14. Vorrichtung nach einem der Ansprüche 1 bis 13, bei der die elastische Hülse (2) eine Dicke von weniger als 1 mm aufweist.

## Claims

1. An orthopedic device (1) for the treatment of Hallux Valgus (Ha), the device being compatible with shoes, consisting essentially of a an elastic sleeve intended to be worn on the foot (P) to exert a localized restraining effect on the metatarsals (M1, M2, M3, M4, M5) of the foot (P), the sleeve comprising:
a main part (3) intended to fit the foot (P) locally on the metatarsals (M1, M2, M3, M4, M5) of the foot,
a distal part (5) for encapsulating the big toe (Go), and
a connecting band (6) between the main part (3) and the distal part (5), intended to be positioned laterally to the foot to be tensioned in order to exert a lateral force (F2) on the big toe (Go) that is directed inwards in relation to the plane of symmetry of the human body and
a pad (4) secured onto the inner wall of the elastic sleeve (2), creating a localized increased thickness, the pad being arranged to be positioned laterally to the foot, locally against the first metatarsal of the big toe (Go) **characterized in that** the pad (4,43) is made of a material (40) having a friction coefficient such that the pad (4, 43) is in contact with the skin of the foot, without slipping, under the pressure of the restraining effect exerted by the sleeve (2)so as to act as an anchor during the application of the lateral force (F2), at least a part of the pad being capable of being placed in different positions along the first metatarsal of the big toe (Go) so as to be able to adjust the tensile force on the connecting band (6).

2. Device according to claim 1, wherein the pad (4, 43) has a thickness between 2mm and 7mm, a length between 4cm and 6cm and a width between 2cm and 4cm.

3. Device according to one of claims 1 to 2, wherein the pad (43) is secured inside the sleeve on the connecting band (6) and on the main part (3) of the sleeve (2), the pad (43) being made of an elastic material so as to be capable of being stretched and keeping its stretch by contact with the skin of the foot, without slipping, so as to contribute to exerting the lateral force (F2) on the big toe (Go).

4. Device according to one of claims 1 to 3, wherein the material of the pad (4, 43, 44) is neoprene or silicone, or silicone gel.

5. Device according to claim 4, wherein the silicone gel forming the material of the pad (4, 43) in contact with the skin comprises an active ingredient capable of being transmitted by contact to the skin.

6. Device according to one of claims 1 to 5, having a proximal part (7) intended to press against the rear part of the foot (P) to hold the elastic sleeve (2) on the foot.

7. Device according to claim 6, wherein the proximal part (7) is produced by a loop (70) of the elastic sleeve (2), intended to be positioned around the ankle of the foot (P).

8. Device according to one of claims 1 to 7, wherein the pad (4) is secured to the elastic sleeve (2) permanently or using removable fixing means.

9. Device according to one of claim 8, wherein the removable fixing means comprise a pocket formed in the elastic sleeve (2), capable of receiving the pad (4) in a removable manner, or comprise a loop-and-hook fixing system between the pad (4) and the elastic sleeve.

10. Device according to one of claims 1 to 9, wherein the distal part (5) is preformed so as to encapsulate the big toe without being in contact with the skin of the big toe in lateral edge regions (OL1, OL2) of the nail of the big toe, in the absence of tensile force exerted on the connecting band (6), so as to limit the pressure exerted by the orthosis on the skin of the big toe in the lateral edge regions of the nail, in the presence of a tensile force exerted on the connecting band (6).

11. Device according to one of claims 1 to 10, wherein the elastic sleeve (2) is made of an elastic textile such as elasthane.

12. Device according to one of claims 1 to 11, wherein the distal part (5) and the connecting band (6) are made of an elastic textile that is less stiff than the textile forming the main part (3) of the sleeve (2).

13. Device according to claim 12, wherein the elastic sleeve (2) is made from a textile pattern after sewing operations.

14. Device according to one of claims 1 to 13, wherein the elastic sleeve (2) has a thickness of less than 1 mm.
